# EUROPÄISCHE PATENTSCHRIFT

(11) **EP 4 018 838 B1**
(45) Veröffentlichungstag und Bekanntmachung des Hinweises auf die Patenterteilung: **22.05.2024**
(21) Anmeldenummer: 20216997.5
(22) Anmeldetag: 23.12.2020
(51) Int. Cl.: A23L 2/38, G01N 21/85, G01N 33/14

(54) **ANLAGE ZUR HERSTELLUNG EINES WÄSSRIGEN LEBENSMITTELS, VERWENDUNG EINES FT-NIR-SPEKTROMETERS IN EINER ANLAGE ZUR HERSTELLUNG EINES WÄSSRIGEN LEBENSMITTELS, MISCHVORRICHTUNG FÜR EINE ANLAGE ZUR HERSTELLUNG EINES WÄSSRIGEN LEBENSMITTELS SOWIE VERFAHREN ZUR HERSTELLUNG VON WÄSSRIGEN LEBENSMITTELN**
PLANT FOR THE PRODUCTION OF AQUEOUS FOODSTUFF, USE OF A FT-NIR- SPECTROMETER IN A PLANT FOR THE PRODUCTION OF AQUEOUS FOODSTUFF, MIXING DEVICE FOR A PLANT FOR THE PRODUCTION OF AQUEOUS FOODSTUFF AND METHOD FOR THE PREPARATION OF AQUEOUS FOODSTUFF
INSTALLATION DE FABRICATION D'UN ALIMENT AQUEUX, UTILISATION D'UN SPECTROMÈTRE FT-NIR DANS UN SYSTÈME POUR LA PRODUCTION D'UN ALIMENT AQUEUX, DISPOSITIF DE MÉLANGE POUR UNE INSTALLATION POUR LA PRODUCTION D'UN ALIMENT AQUEUX AINSI QUE PROCÉDÉ DE FABRICATION DE DENRÉES ALIMENTAIRES AQUEUSES

(43) Veröffentlichungstag der Anmeldung: 29.06.2022
(73) Patentinhaber: Red Bull GmbH, 5330 Fuschl am See (AT)
(72) Erfinder: Nachbagauer, Josef, Fuschl am See (AT); Rinderer, Christian, Fuschl am See (AT); Witwer, Lukas, Fuschl am See (AT)
(74) Vertreter: Metten, Karl-Heinz

(56) Entgegenhaltungen:
- EP-A1- 2 284 517
- DE-A1-102016 109 472
- DE-U1-202007 006 508
- US-A1- 2011 195 161

## Beschreibung

Die vorliegende Erfindung betrifft eine Anlage zur Herstellung eines wässrigen Lebensmittels, insbesondere Getränks wie Energy- oder Softdrink. Ferner betrifft die Erfindung ein Verfahren zur, insbesondere kontinuierlichen, Herstellung von wässrigen Lebensmitteln, insbesondere Getränken. Schließlich betrifft die Erfindung die Verwendung eines Infrarotspektrometers zur Detektion von einem oder mehreren Inhaltsstoffen für wässrige Lebensmittel in einer Anlage zur Herstellung einer Vielzahl an mit einem wässrigen Lebensmittel befüllten Gebinden bzw. zur in-line-Überwachung von einem oder mehreren Inhaltsstoffen für wässrige Lebensmittel, in einer Anlage zur Herstellung einer Vielzahl an mit einem wässrigen Lebensmittel befüllten Gebinden.

Getränke wie Soft- und Energy-Drinks werden schon seit längerem in Highspeed-Produktionsanlagen abgefüllt und versandfertig gemacht. Beispielsweise lassen sich heutzutage ohne weiteres mehrere tausend Gebinde pro Stunde abfüllen. Bei der Herstellung von Getränken, umfassend z.B. das Zusammenführen und Mischen von Einzelkomponenten, die Prozessierung der Ausgangs- bzw. Zwischenprodukte, die Abfüllung von Endprodukten in geeignete Gebinde wie Flaschen oder Dosen und die Durchführung eines Pasteurisierungsschritt, sind vielfältigste hohe Qualitäts- und Hygienestandards fortwährend einzuhalten. Diese Vorgaben stellen hohe Anforderungen sowohl an das Personal, dessen Ausund Weiterbildung wie auch an die Kontrolle und Wartung der eingesetzten Apparaturen. Stellt man im Nachhinein eine nicht spezifikationskonforme Charge fest, sind mitunter große Mengen an Produkt aufwendig zu entsorgen. Um die Einhaltung von Qualitätsstandards prüfen und dokumentieren zu können, werden regelmäßig stichprobenartig off-line Labortests durchgeführt. Diese Vorgehensweise liefert zwar sehr exakte Analyseergebnisse, ist jedoch aufwendig und liefert in Bezug auf den Zeitpunkt der Probennahme stark zeitversetzte Resultate. Im Fall nicht spezifikationskonformer Produkte ist eine große Zahl an befüllten Gebinden zu entsorgen.

Aus der DE 20 2007 006 508 U1 ist eine Vorrichtung zur Bestimmung eines Mischungsverhältnisses eines aus zwei oder mehreren Einzelkomponenten bestehenden Mediums bekannt. Diese Vorrichtung umfasst eine Absorptionsmesszelle, der das zu messende Medium zuführbar ist. Hierbei hat der Absorptionsmesszelle eine von einer Strahlungsquelle erzeugte, das Medium durchdringende elektromagnetische Strahlung zuführbar zu sein. Die Vorrichtung hat ferner ein Spektrometer zu umfassen, durch das das Absorptionsspektrum des zu messenden Mediums erfassbar ist. Auch hat das Ausgangssignal des Spektrometers einer Auswerteinheit zuführbar zu sein. Mit Hilfe der aus dem gemessenen Absorptionsspektrum des Mediums und der vorbestimmten Absorptionsspektren des Mediums oder der das Medium ausbildenden Einzelkomponenten soll sich chemometrisch das Mischungsverhältnis dieser Einzelkomponenten bestimmen lassen.

Der vorliegenden Erfindung hat daher die Aufgabe zugrunde gelegen, eine Anlage zur Herstellung eines wässrigen Lebensmittels, insbesondere eine Anlage für die Herstellung einer Vielzahl an mit einem wässrigen Lebensmittel befüllten Gebinden zur Verfügung zu stellen, die nicht mehr mit den vorangehend geschilderten Nachteilen behaftet ist und die insbesondere eine schnelle und zuverlässige Überprüfung der Qualität bei der Herstellung von Getränken und/oder eine, insbesondere kontinuierliche, Überwachung von für die Herstellung von Getränken wesentlichen Parametern gewährleistet.

Demgemäß wurde eine Anlage zur Herstellung einer Vielzahl an mit einem wässrigen Lebensmittel, insbesondere Getränk wie Energy- oder Softdrink, befüllten Gebinden gemäß den Merkmalen des Patentanspruchs 1 gefunden, umfassend
a) mindestens eine Mischvorrichtung mit einem Mischbehälter und
b) mindestens eine Zuleitung für fluide Medien, insbesondere flüssige Medien, bestehend aus oder enthaltend, insbesondere enthaltend, mindestens einen Inhaltsstoff oder eine Vielzahl an Inhaltsstoffen, für wässrige Lebensmittel, zu dem Mischbehälter,
c) eine Ableitung für ein flüssiges Medium, enthaltend das in der Mischvorrichtung gemischte wässrige Lebensmittel, aus dem Mischbehälter sowie
d) mindestens ein FT-NIR-Spektrometer, eingerichtet und ausgelegt zur in-line-Detektion von Inhaltsstoffen für wässrige Lebensmittel in der mindestens einen Zuleitung für fluide, insbesondere flüssige, Medien und/oder
e) mindestens ein FT-NIR-Spektrometer, eingerichtet und ausgelegt zur in-line-Detektion von Inhaltsstoffen für wässrige Lebensmittel in der mindestens einen Ableitung

Ferner ist erfindungsgemäß in einer geeigneten Ausführungsform eine Anlage zur Herstellung eines wässrigen Lebensmittels, insbesondere Getränks wie Energy- oder Softdrink, gemäß den Merkmalen des Patentanspruchs 1 vorgesehen, umfassend
a) mindestens eine Mischvorrichtung mit einem Mischbehälter und
b) mindestens eine Zuleitung für fluide Medien, insbesondere flüssige Medien, bestehend aus oder enthaltend, insbesondere enthaltend, mindestens einen Inhaltsstoff oder eine Vielzahl an Inhaltsstoffen, für wässrige Lebensmittel, zu dem Mischbehälter,
c) eine Ableitung für ein flüssiges Medium, enthaltend das in der Mischvorrichtung gemischte wässrige Lebensmittel, aus dem Mischbehälter und
d) eine Bypass-Leitung, die, insbesondere benachbart zur Mischvorrichtung, von der Ableitung ausgeht, und die förderstromabwärts, insbesondere benachbart zur Abfüllvorrichtung, in die Ableitung mündet,
   sowie
e) mindestens ein FT-NIR-Spektrometer, eingerichtet und ausgelegt zur in-line-Detektion von Inhaltsstoffen für wässrige Lebensmittel in der mindestens einen Zuleitung für fluide Medien und/oder
f) mindestens ein FT-NIR-Spektrometer, eingerichtet und ausgelegt zur in-line-Detektion von Inhaltsstoffen für wässrige Lebensmittel in der mindestens einen Ableitung und/oder
g) mindestens ein FT-NIR-Spektrometer, eingerichtet und ausgelegt zur inline-Detektion von Inhaltsstoffen für wässrige Lebensmittel in der Bypass-Leitung.

Mit der erfindungsgemäßen apparativen Einbindung eines FT-NIR-Spektrometers in eine Anlage zur Herstellung einer Vielzahl an mit einem wässrigen Lebensmittel befüllten Gebinden gelingt die qualitative wie auch die quantitative in-line-Überwachung von einem oder mehreren Inhaltsstoffen für wässrige Lebensmittel, und zwar überraschender Weise sogar über einen sehr breiten Konzentrationsbereich. Demgemäß sind sowohl die Haupt- wie auch Nebenbestandteile eines flüssigen Lebensmittels detektier- und überwachbar.

Bei den in der erfindungsgemäßen Anlage zum Einsatz kommenden Infrarotspektrometern handelt es sich um Fourier-Transformations (FT)-NIR-Spektrometer. Das mindestens eine FT-NIR-Spektrometer umfasst dabei mindestens einen Lichtleiter und eine Transmissionsprozesssonde, eingerichtet und ausgelegt, um mit dem mindestens einen fluiden, insbesondere flüssigen, Medium, bestehend aus oder enthaltend den mindestens einen Inhaltsstoff, oder mit dem wässrigen Lebensmittel in Kontakt zu treten. In einer praktischen Ausgestaltung geht der mindestens eine Lichtleiter in die Transmissionsprozesssonde über bzw. knüpft an diese an. Diese Transmissionsprozesssonde, z.B. in stabförmiger Ausgestaltung, kann als Bestandteil des Infrarotspektrometers in den mit dem wässrigen Lebensmittel befüllten Behälter sowie insbesondere auch in die Zu-, Ab- oder Transferleitungen hineinragen, um die für die qualitative und/oder quantitative Analyse erforderliche Wechselwirkung der Inhaltsstoffe mit der Infrarotstrahlung herbeizuführen.

Das mindestens eine FT-NIR-Spektrometer umfasst insbesondere eine Datenverarbeitungseinrichtung, eingerichtet und ausgelegt, die infrarotspektroskopisch ermittelten Daten der jeweiligen Inhaltsstoffe, insbesondere Getränkeinhaltsstoffe, der fluiden, insbesondere flüssigen, Medien oder des wässrigen Lebensmittels zu speichern und auszuwerten, insbesondere abzugleichen mit in der Datenverarbeitungseinrichtung hinterlegten Referenzdaten für den oder die jeweiligen Inhaltsstoffe. Hierbei kann die Auswertung sowohl qualitativ als auch quantitativ erfolgen. Das mindestens eine FT-NIR-Spektrometer ist dabei insbesondere ausgelegt und eingerichtet, den Gehalt an zwei oder mehr Inhaltstoffen, insbesondere Getränkeinhaltstoffen, simultan zu ermitteln. Insbesondere unter Einsatz der Datenverarbeitungseinrichtung gelingt es dabei auch, den mindestens einen Inhaltsstoff in dem flüssigen Medium oder in dem wässrigen Lebensmittel quantitativ zu erfassen.

Von weiterem Vorteil der erfindungsgemäßen Anlage ist auch, dass unter Einbindung des FT-NIR-Spektrometers sich der mindestens eine Inhaltsstoff für wässrige Lebensmittel, insbesondere die Vielzahl an Inhaltsstoffen, quasi-kontinuierlich detektieren lassen, bevorzugt in wiederkehrenden Messintervallen im Bereich von 5 bis 90 Sekunden und besonders bevorzugt im Bereich von 10 bis 20 Sekunden. Bedingt durch die sehr kurzen Messintervalle kann der Herstellprozess im Wesentlichen nahtlos überwacht werden. Mit Hilfe der erfindungsgemäßen Anlage können infrarotspektroskopisch für einen Messzeitpunkt ermittelten Daten der jeweiligen Inhaltsstoffe, insbesondere Getränkeinhaltsstoffe, der fluiden, insbesondere flüssigen, Medien oder des wässrigen Lebensmittels gestützt durch eine Datenverarbeitungseinrichtung einer Charge bzw. Abfolge an aufeinander folgend mit dem flüssigen Lebensmittel abgefüllten Gebinden sowie insbesondere einem einzelnen mit dem flüssigen Lebensmittel befüllten Gebinde zugeordnet und beispielsweise in einen auf den Gebinde bzw. dem Gebinde aufgebrachten Code eingelesen werden. Insbesondere können die infrarotspektroskopisch ermittelten und einem Messzeitpunkt zugeordneten Daten der jeweiligen Inhaltsstoffe einem in der Abfüllvorrichtung mit dem wässrigen Lebensmittel, insbesondere der wässrigen Getränkemischung, befüllten Gebindegrundkörper oder einer Mehrzahl an aufeinander folgend mit dem wässrigen Lebensmittel, insbesondere der wässrigen Getränkemischung, befüllten Gebindegrundkörpern unter Einsatz der Datenverarbeitungseinrichtung zugeordnet werden. Damit gelingt über einen auf dem Gebinde angebrachten Produktcode auch noch nach dem Verkauf eines Gebindes eine zuverlässige Ermittlung der konkret verwendeten Inhaltsstoffe und Mengen derselben, mithin eine nachträgliche Bestätigung einer spezifikationskonformen Herstellung.

In einer möglichen Ausführungsform kann dabei mindestens ein FT-NIR-Spektrometer (Komponente h)) vorgesehen sein, das eingerichtet und ausgelegt ist zur in-line-Detektion von Inhaltsstoffen für wässrige Lebensmittel in der Mischvorrichtung selber.

Die erfindungsgemäße Anlage, insbesondere als Anlage für die Herstellung von Getränken, umfasst in einer zweckmäßigen Ausgestaltung ferner eine Abfüllvorrichtung (Komponente i)), die über die Ableitung mit der Mischvorrichtung verbunden oder verbindbar ist, ausgelegt und eingerichtet zum Einfüllen des wässrigen Lebensmittels, insbesondere der wässrigen Getränkemischung, erhalten oder erhältlich mit der Mischvorrichtung, in eine Vielzahl an Gebindegrundkörpern, bevorzugt Getränkegebindegrundkörpern, besonders bevorzugt Getränkedosengrundkörpern, jeweils enthaltend einen Gebindeboden, eine Gebindewandung und eine Einfüllöffnung.

In einer weiteren zweckmäßigen Ausgestaltung ist die erfindungsgemäße Anlage ausgestattet mit der Bypass-Leitung (Komponente d)), die benachbart zur Mischvorrichtung von der Ableitung ausgeht und die förderstromabwärts benachbart zur Abfüllvorrichtung in die Ableitung mündet. Dabei kann in einer zweckmäßigen Ausführungsform in dieser Bypass-Leitung mindestens ein FT-NIR-Spektrometer (Komponente g)) vorgesehen sein, eingerichtet und ausgelegt zur inline-Detektion von Inhaltsstoffen für wässrige Lebensmittel.

Die erfindungsgemäße Anlage ist in einer zweckmäßigen Ausführungsform mit einer Ableitung und einer Bypass-Leitung ausgestattet, die, insbesondere benachbart zur Mischvorrichtung, von der Ableitung ausgeht, und die förderstromabwärts, insbesondere benachbart zur Abfüllvorrichtung, in die Ableitung mündet, sowie mindestens eine Messzelle, die zwischen dem Ausgang und der Mündung der Bypass-Leitung in der Ableitung oder in der Bypass-Leitung, insbesondere in der Ableitung, mit dem wässrigen Lebensmittel in Kontakt tritt.

In einer weiteren zweckmäßigen Ausgestaltung ist die erfindungsgemäße Anlage ferner ausgestattet mit mindestens eine Verschlussvorrichtung (Komponente j)), ausgelegt und eingerichtet zum, insbesondere dichten, Verschließen des mit der Abfüllvorrichtung befüllten Gebindegrundkörpers, bevorzugt Getränkegebindegrundkörpers, besonders bevorzugt des befüllten Getränkedosengrundkörpers, mit einem Gebindeverschluss, insbesondere einem Getränkedosendeckel.

Ein Gegenstand der vorliegenden Erfindung ist damit auch eine solche Vorrichtung, geeignet als Bestandteil einer Anlage zur Herstellung eines wässrigen Lebensmittels, insbesondere einer Anlage für die Herstellung einer Vielzahl an mit einem wässrigen Lebensmittel befüllten Gebinden, bestehend aus oder umfassend mindestens eine Mischvorrichtung mit einem Mischbehälter, mindestens eine Zuleitung für flüssige Medien, enthaltend mindestens einen Inhaltsstoff, insbesondere eine Vielzahl an Inhaltsstoffen, für wässrige Lebensmittel, zu dem Mischbehälter, eine Ableitung für ein flüssiges Medium, enthaltend das in der Mischvorrichtung gemischte wässrige Lebensmittel, aus dem Mischbehälter sowie mindestens ein FT-NIR-Spektrometer, eingerichtet und ausgelegt zur in-line-Detektion von Inhaltsstoffen für wässrige Lebensmittel in der mindestens einen Zuleitung für flüssige Medien und/oder, insbesondere und, mindestens ein FT-NIR-Spektrometer, eingerichtet und ausgelegt zur in-line-Detektion von Inhaltsstoffen für wässrige Lebensmittel in der mindestens einen Ableitung. Alternativ oder zusätzlich kann dieser Anlagenbestandteil auch eine von der Ableitung ausgehende und in diese wieder mündende Bypass-Leitung aufweisen, welche vorzugsweise mit einem FT-NIR-Spektrometer ausgestattet ist.

Die erfindungsgemäße Anlage, insbesondere als Anlage für die Herstellung von Getränken, ist in einer zweckmäßigen Ausgestaltung ferner dadurch gekennzeichnet, dass die Mischvorrichtung ausgelegt und eingerichtet ist zum Mischen von Inhaltsstoffen, insbesondere Getränkeinhaltsstoffen, unter Erhalt eines, insbesondere carbonisierten, wässrigen Lebensmittels, insbesondere einer Getränkemischung, enthaltend den Mischbehälter sowie eine erste Zuleitung zu diesem Mischbehälter für ein, insbesondere carbonisiertes, wässriges System und eine zweite Zuleitung zu diesem Mischbehälter für mindestens einen ersten Inhaltsstoff, insbesondere Getränkeinhaltsstoff, oder eine erste Inhaltsstoffmischung, insbesondere Getränkeinhaltsstoffmischung, sowie gegebenenfalls eine dritte Zuleitung zu diesem Mischbehälter für mindestens einen Inhaltsstoff, insbesondere zweiten Getränkeinhaltsstoff, oder eine zweite Inhaltsstoffmischung, insbesondere Getränkeinhaltsstoffmischung, sowie gegebenenfalls eine vierte Zuleitung zu diesem Mischbehälter für mindestens einen dritten Inhaltsstoff, insbesondere Getränkeinhaltsstoff, oder eine dritte Inhaltsstoffmischung, insbesondere Getränkeinhaltsstoffmischung und gegebenenfalls mindestens eine weitere Zuleitung zu diesem Mischbehälter für mindestens einen weiteren Inhaltsstoff, insbesondere Getränkeinhaltsstoff, oder mindestens eine weitere Inhaltsstoffmischung, insbesondere Getränkeinhaltsstoffmischung.

Die der Erfindung zugrunde liegende Aufgabe wird demgemäß ferner gelöst durch eine Mischvorrichtung für eine Anlage zur Herstellung eines wässrigen Lebensmittels, insbesondere Getränks wie Energy- oder Softdrink, gemäß den Merkmalen des Patentanspruchs 21, umfassend mindestens einen Mischbehälter; mindestens eine Zuleitung für flüssige Medien, enthaltend mindestens einen Inhaltsstoff, insbesondere eine Vielzahl an Inhaltsstoffen, für wässrige Lebensmittel, zu dem Mischbehälter; eine Ableitung für ein flüssiges Medium, enthaltend das in dem Mischbehälter gemischte wässrige Lebensmittel, aus dem Mischbehälter; sowie mindestens ein FT-NIR-Spektrometer, eingerichtet und ausgelegt zur in-line-Detektion von Inhaltsstoffen für wässrige Lebensmittel in der mindestens einen Zuleitung für flüssige Medien und/oder, insbesondere und, mindestens ein FT-NIR-Spektrometer, eingerichtet und ausgelegt zur in-line-Detektion von Inhaltsstoffen für wässrige Lebensmittel in der mindestens einen Ableitung.

Die erfindungsgemäße Anlage stellt bevorzugt eine Anlage zur High-Speed-Herstellung dar.

In einer weiteren bevorzugten Ausführungsform umfasst die erfindungsgemäße Anlage ferner mindestens eine Anmischvorrichtung, ausgelegt und eingerichtet zum Mischen von Inhaltsstoffen, insbesondere Getränkeinhaltsstoffen, unter Erhalt eines wässrigen Lebensmittelkonzentrats, insbesondere eines Getränkemischungskonzentrats, enthaltend einen Anmischbehälter sowie eine Zuleitung zu diesem Anmischbehälter für ein wässriges System, insbesondere Wasser, und eine Zuleitung zu diesem Anmischbehälter für mindestens einen ersten Inhaltsstoff, insbesondere Getränkeinhaltsstoff, oder eine erste Inhaltsstoffmischung, insbesondere Getränkeinhaltsstoffmischung, sowie gegebenenfalls eine Zuleitung zu diesem Anmischbehälter für mindestens einen zweiten Inhaltsstoff, insbesondere zweiten Getränkeinhaltsstoff, oder eine zweite Inhaltsstoffmischung, insbesondere Getränkeinhaltsstoffmischung, sowie gegebenenfalls eine Zuleitung zu diesem Anmischbehälter für mindestens einen dritten Inhaltsstoff, insbesondere Getränkeinhaltsstoff, oder eine dritte Inhaltsstoffmischung, insbesondere Getränkeinhaltsstoffmischung, sowie gegebenenfalls eine Zuleitung zu diesem Anmischbehälter für mindestens einen vierten Inhaltsstoff, insbesondere Getränkeinhaltsstoff, oder eine vierte Inhaltsstoffmischung, insbesondere Getränkeinhaltsstoffmischung, und mindestens eine Transferleitung für ein flüssiges Medium, enthaltend das oder bestehend aus dem in der Anmischvorrichtung gemischte wässrige Lebensmittelkonzentrat, insbesondere Getränkemischungskonzentrat, und gegebenenfalls mindestens eine Bypass-Leitung, die, insbesondere benachbart zur Anmischvorrichtung, von der Transferleitung ausgeht und die in die Anmischvorrichtung mündet. Diese Anlagenausführungsform ist darüber hinaus ausgestattet mit mindestens einem FT-NIR-Spektrometer, eingerichtet und ausgelegt zur inline-Detektion von Inhaltsstoffen für wässrige Lebensmittel in der Transferleitung und/oder mit mindestens einem FT-NIR-Spektrometer, eingerichtet und ausgelegt zur inline-Detektion von Inhaltsstoffen für wässrige Lebensmittel in der Bypass-Leitung. Bevorzugt wird hierbei auf ein FT-NIR-Spektrometer, eingerichtet und ausgelegt zur inline-Detektion von Inhaltsstoffen für wässrige Lebensmittel in der Transferleitung zurückgegriffen. Demgemäß kann das wässrige System in dem Anmischbehälter und/oder in der Transferleitung von dem Anmischbehälter und/oder in der Bypass-Leitung des Anmischbehälters und/oder der erste und/oder zweite Inhaltsstoff, insbesondere Getränkeinhaltsstoff, oder die erste und/oder zweite Inhaltsstoffmischung, insbesondere Getränkeinhaltsstoffmischung, in der jeweiligen Zuleitung zu dem Anmischbehälter sowie gegebenenfalls der dritte und/oder vierte Inhaltsstoff, insbesondere Getränkeinhaltsstoff, oder die dritte und/oder vierte Inhaltsstoffmischung, insbesondere Getränkeinhaltsstoffmischung, in der jeweiligen Zuleitung zu dem Anmischbehälter mit dem dem Anmischbehälter, der Transferleitung, der Bypass-Leitung bzw. der jeweiligen Zuleitung zugeordneten Infrarotspektrometer auf den Gehalt an einen oder mehreren Inhaltsstoffen, insbesondere Getränkeinhaltsstoffen, analysiert werden.

Die der Erfindung zugrunde liegende Aufgabe wird demgemäß ferner gelöst durch eine Anmischvorrichtung gemäß den Merkmalen des Patentanspruchs 22, ausgelegt und eingerichtet zum Mischen von Inhaltsstoffen, insbesondere Getränkeinhaltsstoffen, unter Erhalt eines wässrigen Lebensmittelkonzentrats, insbesondere eines Getränkemischungskonzentrats, enthaltend einen Mischbehälter sowie eine Zuleitung zu diesem ersten Mischbehälter für ein wässriges System, insbesondere

Wasser, und eine Zuleitung zu diesem ersten Mischbehälter für mindestens einen ersten Inhaltsstoff, insbesondere Getränkeinhaltsstoff, oder eine erste Inhaltsstoffmischung, insbesondere Getränkeinhaltsstoffmischung, sowie gegebenenfalls eine Zuleitung zu diesem Mischbehälter für mindestens einen zweiten Inhaltsstoff, insbesondere zweiten Getränkeinhaltsstoff, oder eine zweite Inhaltsstoffmischung, insbesondere Getränkeinhaltsstoffmischung, sowie gegebenenfalls eine Zuleitung zu diesem Mischbehälter für mindestens einen dritten Inhaltsstoff, insbesondere Getränkeinhaltsstoff, oder eine dritte Inhaltsstoffmischung, insbesondere Getränkeinhaltsstoffmischung, sowie gegebenenfalls eine Zuleitung zu diesem Mischbehälter für mindestens einen vierten Inhaltsstoff, insbesondere Getränkeinhaltsstoff, oder eine vierte Inhaltsstoffmischung, insbesondere Getränkeinhaltsstoffmischung, und mindestens eine Ableitung für ein flüssiges Medium, enthaltend das in der Anmischvorrichtung gemischte wässrige Lebensmittelkonzentrat, insbesondere Getränkemischungskonzentrat, und gegebenenfalls mindestens eine Bypass-Leitung, die, insbesondere benachbart zur Anmischvorrichtung, von der ersten Ableitung ausgeht, und die in der Anmischvorrichtung mündet, sowie mindestens ein FT-NIR-Spektrometer, eingerichtet und ausgelegt zur inline-Detektion von Inhaltsstoffen für wässrige Lebensmittel in der Ableitung und/oder mindestens ein FT-NIR-Spektrometer, eingerichtet und ausgelegt zur inline-Detektion von Inhaltsstoffen für wässrige Lebensmittel in der ersten Bypass-Leitung.

In einer besonders zweckmäßigen Weiterentwicklung der erfindungsgemäßen Anlage ist in diese ferner ein Massenspektrometer, bevorzugt ein qTOF-Massenspektrometer und besonders bevorzugt eine ESI-qTOF-Massenspektrometer, integriert, enthaltend eine Probenleitung zu diesem Massenspektrometer, eingerichtet und ausgelegt zur Detektion von Inhaltsstoffen, insbesondere Getränkeinhaltsstoffen, in wässrigen Lebensmitteln oder in Vorstufen hiervon. Hierbei können Probenvolumina insbesondere auch an den Stellen entnommen und anschließend analysiert werden, wie vorangehend für die geschilderten infrarotspektroskopischen Untersuchungen ausgeführt. Bevorzugt werden dabei über eine Probenentnahmeleitung, die von der Verbindungsleitung zwischen der Misch- und der Abfüllvorrichtung abgeht, dem Massenspektrometer, insbesondere auch verdünnte, Probenvolumina, insbesondere intervallweise, zugeführt. Durch die Einbindung eines Massenspektrometers in die erfindungsgemäße Anlage lässt sich die Messgenauigkeit über das gesamte Spektrum relevanter Inhaltsstoffe weiter verbessern und die Qualität der Überwachung nochmals erhöhen.

Die der Erfindung zugrunde liegende Aufgabe wird ebenfalls gelöst durch ein Verfahren zur, insbesondere kontinuierlichen, Herstellung von wässrigen Lebensmitteln, insbesondere Getränken, gemäß den Merkmalen des Patentanspruchs 12, umfassend
- die Zurverfügungstellung einer Anlage, insbesondere High-Speed-Produktionsanlage, gemäß einem oder mehreren der vorangehenden Patentansprüche,
- die Zurverfügungstellung von Gebindegrundkörpern, bevorzugt Getränkegebindegrundkörpern, besonders bevorzugt Getränkedosengrundkörpern, enthaltend einen Gebindeboden, eine Gebindewandung und eine Einfüllöffnung,
- die Zurverfügungstellung eines Gebindeverschlusses, insbesondere eines Getränkedosendeckels,
- die Zurverfügungstellung eines, insbesondere carbonisierten, wässrigen Systems,
- die Zurverfügungstellung von mindestens einem, insbesondere einer Vielzahl an Inhaltsstoffen, insbesondere Getränkeinhaltsstoffen, sowie
- gegebenenfalls die Zurverfügungstellung von mindestens einem, insbesondere carbonisierten, wässrigen Süßungsmittelkonzentrat,
- das Mischen des wässrigen Systems mit dem mindestens einen oder der Vielzahl an Inhaltsstoffen sowie gegebenenfalls dem wässrigen Süßungsmittelkonzentrat in der Mischvorrichtung unter Erhalt eines wässrigen Lebensmittels, insbesondere einer wässrigen Getränkemischung,
- das Einfüllen des, insbesondere carbonisierten, wässrigen Lebensmittels in die Vielzahl an Gebindegrundkörpern und
- das Verschließen der mit dem wässrigen Lebensmittel befüllten Gebindegrundkörper mit einem Gebindeverschluss mittels der Verschlussvorrichtung.

Das erfindungsgemäße Verfahren hat ferner zu umfassen
- die, insbesondere quantitative, inline-Detektion des mindestens einen Inhaltstoffs in einem in der mindestens einen Zuleitung transportieren flüssigen Mediums durch das mindestens eine, dieser Zuleitung zugeordnete FT-NIR-Spektrometer und/oder
- die, insbesondere quantitative, inline-Detektion des mindestens einen Inhaltstoffs in der Ableitung für flüssige Medien durch das mindestens eine, dieser Ableitung zugeordnete FT-NIR-Spektrometer und/oder
- die, insbesondere quantitative, inline-Detektion des mindestens einen Inhaltstoffs des in der Mischvorrichtung vorliegenden wässrigen Lebensmittels durch das mindestens eine, dieser Mischvorrichtung zugeordnete FT-NIR-Spektrometer.

Bevorzugt wird dabei die inline-Detektion des mindestens einen Inhaltstoffs mittels FT-NIR-Spektrometrie in der Ableitung für flüssige Medien vorgenommen. Auf diese Weise ist es möglich, die Qualität des wässrigen Lebensmittels vor der Abfüllung in einzelne Gebinde zu überwachen. Die inline-Detektion kann dabei sowohl für die qualitative wie auch insbesondere die quantitative Analyse des/der Inhaltsstoffe genutzt werden.

Die, insbesondere quantitative, inline-Detektion des mindestens einen Inhaltstoffs mittels FT-NIR-Spektrometrie in der mindestens einen Zuleitung zu dem Mischbehälter wird insbesondere vorgesehen, vorzugsweise zur infrarotspektroskopischen Überwachung der Ableitung, bei Einbindung der Anmischvorrichtung in die erfindungsgemäße Anlage. Hierdurch wird eine zuverlässige Kontrolle der Qualität des aus der Anmischvorrichtung entstammenden Konzentrats sichergestellt, insbesondere auch durch einen Abgleich mit den infrarotspektroskopisch für das bei Verlassen des Anmischbehälters für das darin hergestellte Konzentrat ermittelten Daten. Hierbei können die infrarotspektroskopisch ermittelten, insbesondere quantitativen, Daten für einen oder mehrere Inhaltsstoffe, insbesondere Getränkeinhaltsstoffe, einer Charge des über die Transferleitung dem Anmischbehälter entnommenen wässrigen Lebensmittelkonzentrats, insbesondere eines Getränkemischungskonzentrats, mit den infrarotspektroskopisch ermittelten, insbesondere quantitativen, Daten für einen oder mehrere Inhaltsstoffe, insbesondere Getränkeinhaltsstoffe, dieser Charge vor oder bei Eintritt in den Mischbehälter der Mischvorrichtung über die Zuleitung verglichen werden. Auf diese Weise kann zudem sehr zuverlässig eine Identitätskontrolle der prozessierten flüssigen Medien implementiert werden. Vielfach ist es hinreichend, mit Hilfe der ermittelten Daten eine qualitative Auswertung dahingehend vorzunehmen, ob sich das analysierte System einer bestimmten, insbesondere vorgegebenen, Kategorie, z.B. Energy-Drink, Soft-Drink, Cola-Drink oder dergleichen, zuordnen lässt.

Die vorangehend geschilderte doppelte Prüfung mittels Infrarotspektroskopie - einmal direkt nach Verlassen des Anmischbehälters, ein weiteres Mal unmittelbar von Eintritt des wässrigen Konzentrats in den Mischbehälter - gestattet es, das in der Anmischvorrichtung erhaltene wässrige Konzentrat zwischenzulagern, bevor es der Mischvorrichtung zugeführt wird. Dies ermöglicht wiederum, in bestimmten Grenzen einen Materialpuffer anzulegen, wodurch ein kontinuierlicher Herstellprozess umfassend die Herstellung des wässrigen Lebensmittels und dessen Abfüllung in Gebinde im Wesentlichen komplikationsfrei aufrechterhalten werden kann.

Bei dem erfindungsgemäßen Verfahren wird das wässrige Lebensmittel, insbesondere Getränk, in der Mischvorrichtung und/oder der Ableitung mit dem der Mischvorrichtung bzw. der Ableitung zugeordneten Infrarotspektrometer bevorzugt auf den Gehalt an Zucker, insbesondere Saccharose, Glucose und/oder Fructose, Zitronensäure, Coffein, Taurin und/oder artifiziellen Süßungsmitteln, insbesondere Acesulfam Kund/oder Aspartam, analysiert, insbesondere quantitativ bestimmt. Von besonderem Vorteil ist dabei auch, dass sich zwei oder mehr der Inhaltsstoffe simultan bestimmen lassen.

Die mit dem erfindungsgemäßen Verfahren infrarotspektroskopisch ermittelten, insbesondere quantitativen, Daten der jeweiligen Inhaltsstoffe, insbesondere Getränkeinhaltsstoffe, der flüssigen Medien oder des wässrigen Lebensmittels werden zweckmäßigerweise in einer Datenverarbeitungseinrichtung gespeichert. Hierbei hat es sich als sehr praktikabel erwiesen, diese infrarotspektroskopisch ermittelten Daten der jeweiligen Inhaltsstoffe, insbesondere Getränkeinhaltsstoffe, der flüssigen Medien oder des wässrigen Lebensmittels in der Datenverarbeitungseinrichtung dem jeweiligen Messzeitpunkt zuzuordnen.

Indem bekannt ist, welche Inhaltsstoffe für ein wässriges Lebensmittel eingesetzt werden, können die infrarotspektroskopisch für einen Messzeitpunkt ermittelten Daten der jeweiligen Inhaltsstoffe, insbesondere Getränkeinhaltsstoffe, der flüssigen Medien oder des wässrigen Lebensmittels in der Datenverarbeitungseinrichtung mit hinterlegten Soll-Bereichswerten abgeglichen werden. Mit dem erfindungsgemäßen Verfahren gelingt damit sowohl eine quantitative wie auch eine qualitative Analyse der jeweiligen Inhaltsstoffe.

Indem mit dem erfindungsgemäßen Verfahren eine kontinuierliche bzw. quasikontinuierliche Kontrolle des Gehalts an Inhaltsstoffen ermöglicht wird, kann mit diesem Verfahren auch regelnd in den laufenden Herstellprozess eingegriffen werden. So kann insbesondere auch der Zutritt des wässrigen Lebensmittels, insbesondere Getränkemischung, zu der Abfüllvorrichtung mit Hilfe eines, insbesondere mittels automatischer Aktivierung eines, Umlenkventils in der Ableitung, verhindert werden, sobald die infrarotspektroskopisch ermittelten, insbesondere quantitativen, Daten für mindestens einen Inhaltsstoff, insbesondere Getränkeinhaltsstoff, des wässrigen Lebensmittels außerhalb seines hinterlegten Soll-Bereichswerts liegt, so dass das wässrige Lebensmittel nicht in die Abfüllvorrichtung gelangt. Alternativ kann ein Umlenkventil in der Ableitung geschlossen bleiben oder werden, solange bzw. sobald die infrarotspektroskopisch ermittelten, insbesondere quantitativen, Daten für sämtliche Inhaltsstoffe, insbesondere Getränkeinhaltsstoffe, des wässrigen Lebensmittels innerhalb ihrer jeweiligen hinterlegten Soll-Bereichswerte liegen.

In einer bevorzugten Ausführungsform des erfindungsgemäßen Verfahrens kann zwecks Erhalts einer optimierten Verfahrensführung auch vorgesehen sein, dass,
sollten die infrarotspektroskopisch ermittelten, insbesondere quantitativen, Daten für einen oder mehrere Inhaltsstoffe, insbesondere Getränkeinhaltsstoffe, des in der mindestens einen Zuleitung transportierten flüssigen Mediums unterhalb der hinterlegten Soll-Bereichswerte liegen, man die Konzentration des oder der Inhaltsstoffe in dem flüssigen Medium, das in der mindestens einen Zuleitung transportiert wird, erhöht, bis die infrarotspektroskopisch ermittelten Daten innerhalb der hinterlegten Soll-Bereichswerte liegen, oder dass, sollten die infrarotspektroskopisch ermittelten, insbesondere quantitativen, Daten für einen oder mehrere Inhaltsstoffe, insbesondere Getränkeinhaltsstoffe, des in der mindestens einen Zuleitung transportierten flüssigen Mediums oberhalb der hinterlegten Soll-Bereichswerte liegen, man die Konzentration des oder der Inhaltsstoffe in dem flüssigen Medium, das in der mindestens einen Zuleitung transportiert wird, erniedrigt, bis die infrarotspektroskopisch ermittelten Daten innerhalb der hinterlegten Soll-Bereichswerte liegen.

Alternativ sowie insbesondere zusätzlich kann vorgesehen sein, dass, sollten die infrarotspektroskopisch ermittelten, insbesondere quantitativen, Daten für einen oder mehrere Inhaltsstoffe, insbesondere Getränkeinhaltsstoffe, des in der Mischvorrichtung vorliegenden wässrigen Lebensmittels unterhalb der hinterlegten Soll-Bereichswerte liegen, man die Konzentration des oder der Inhaltsstoffe in dem in der Mischvorrichtung vorliegenden wässrigen Lebensmittel erhöht, bis die infrarotspektroskopisch ermittelten Daten innerhalb der hinterlegten Soll-Bereichswerte liegen, oder dass, sollten die infrarotspektroskopisch ermittelten, insbesondere quantitativen, Daten für einen oder mehrere Inhaltsstoffe, insbesondere Getränkeinhaltsstoffe, des in der Mischvorrichtung vorliegenden wässrigen Lebensmittels oberhalb der hinterlegten Soll-Bereichswerte liegen, man die Konzentration des oder der Inhaltsstoffe in dem in der Mischvorrichtung vorliegenden wässrigen Lebensmittel erniedrigt, bis die infrarotspektroskopisch ermittelten Daten innerhalb der hinterlegten Soll-Bereichswerte liegen.

Mit der erfindungsgemäßen Anlage und dem erfindungsgemäßen Verfahren können durch Einbindung mindestens eines FT-NIR-Infrarotspektrometers ein oder mehrere Inhaltsstoffe für wässrige Lebensmittel, insbesondere Getränke, in einer Anlage zur Herstellung einer Vielzahl an mit einem wässrigen Lebensmittel, insbesondere Getränk, befüllten Gebinden, insbesondere Getränkegebinden, insbesondere quantitativ, detektiert werden, bei Einsatz mehrerer Inhaltsstoffe sogar simultan, wodurch auch eine, insbesondere quantitative, inline-Überwachung von einem oder mehreren Inhaltsstoffen für wässrige Lebensmittel, insbesondere Getränke, in einer Anlage zur Herstellung einer Vielzahl an mit einem wässrigen Lebensmittel, insbesondere Getränk, befüllten Gebinden, insbesondere Getränkegebinden, möglich ist. Überraschend wurde auch gefunden, dass das erfindungsgemäße Verfahren bei Anlagen zur High-Speed-Herstellung einer Vielzahl an mit einem wässrigen Lebensmittel befüllten Gebinden, insbesondere Getränkegebinden, zuverlässig eingesetzt werden kann. Durch die Einbindung des FT-NIR-Spektrometers in die erfindungsgemäße Anlage als inline-Messwerkzeug lassen sich die Messgenauigkeit über das gesamte Spektrum relevanter Inhaltsstoffe gegenüber bekannten Systemen, die regelmäßig off-line arbeiten, verbessern und die Qualität der Überwachung erhöhen.

## Patentansprüche

1. Anlage zur Herstellung einer Vielzahl an mit einem wässrigen Lebensmittel, insbesondere Getränk, befüllten Gebinden, insbesondere Getränkegebinden, umfassend
a) mindestens eine Mischvorrichtung mit einem Mischbehälter und
b) mindestens eine Zuleitung für fluide Medien, insbesondere flüssige Medien, bestehend aus oder enthaltend, insbesondere enthaltend, mindestens einen Inhaltsstoff oder eine Vielzahl an Inhaltsstoffen für wässrige Lebensmittel, zu dem Mischbehälter,
c) eine Ableitung für ein flüssiges Medium, enthaltend das in der Mischvorrichtung gemischte wässrige Lebensmittel, aus dem Mischbehälter und
d) gegebenenfalls eine Bypass-Leitung, die, insbesondere benachbart zur Mischvorrichtung, von der Ableitung ausgeht, und die förderstromabwärts, insbesondere benachbart zur Abfüllvorrichtung, in die Ableitung mündet, sowie
e) mindestens ein FT-NIR-Spektrometer, eingerichtet und ausgelegt zur in-line-Detektion von Inhaltsstoffen für wässrige Lebensmittel in der mindestens einen Zuleitung für fluide Medien und/oder
f) mindestens ein FT-NIR-Spektrometer, eingerichtet und ausgelegt zur in-line-Detektion von Inhaltsstoffen für wässrige Lebensmittel in der mindestens einen Ableitung und/oder
g) mindestens ein FT-NIR-Spektrometer, eingerichtet und ausgelegt zur inline-Detektion von Inhaltsstoffen für wässrige Lebensmittel in der Bypass-Leitung,
wobei das mindestens eine FT-NIR-Spektrometer mindestens einen Lichtleiter und eine Transmissionsprozesssonde umfasst, eingerichtet und ausgelegt, um mit dem mindestens einen fluiden, insbesondere flüssigen, Medium, bestehend aus oder enthaltend mindestens einen Inhaltsstoff oder die Vielzahl an Inhaltsstoffen, oder mit dem wässrigen Lebensmittel in Kontakt zu treten.

2. Anlage nach Anspruch 1, ferner umfassend
h) mindestens ein Infrarotspektrometer, eingerichtet und ausgelegt zur in-line-Detektion von Inhaltsstoffen für wässrige Lebensmittel in der Mischvorrichtung.

3. Anlage nach Anspruch 1 oder 2, ferner umfassend
i) eine Abfüllvorrichtung, die über die Ableitung mit der Mischvorrichtung verbunden oder verbindbar, ausgelegt und eingerichtet zum Einfüllen des wässrigen Lebensmittels, insbesondere der wässrigen Getränkemischung, erhalten oder erhältlich mit der Mischvorrichtung, in eine Vielzahl an Gebindegrundkörpern, bevorzugt Getränkegebindegrundkörpern, besonders bevorzugt Getränkedosengrundkörpern, jeweils enthaltend einen Gebindeboden, eine Gebindewandung und eine Einfüllöffnung.

4. Anlage nach einem der vorangehenden Ansprüche, **gekennzeichnet durch** die Ableitung und die Bypass-Leitung, die, insbesondere benachbart zur Mischvorrichtung, von der Ableitung ausgeht, und die förderstromabwärts, insbesondere benachbart zur Abfüllvorrichtung, in die Ableitung mündet, sowie eine Messzelle, welche zwischen dem Ausgang und der Mündung der Bypass-Leitung in der Ableitung mit dem wässrigen Lebensmittel in Kontakt tritt.

5. Anlage nach einem der vorangehenden Ansprüche, **dadurch gekennzeichnet, dass** das mindestens eine Infrarotspektrometer eine Datenverarbeitungseinrichtung umfasst, eingerichtet und ausgelegt, die infrarotspektroskopisch ermittelten Daten der jeweiligen Inhaltsstoffe, insbesondere Getränkeinhaltsstoffe, der fluiden, insbesondere flüssigen, Medien oder des wässrigen Lebensmittels zu speichern und, insbesondere qualitativ und/oder quantitative, auszuwerten, insbesondere abzugleichen mit in der Datenverarbeitungseinrichtung hinterlegten Referenzdaten für den oder die jeweiligen Inhaltsstoffe.

6. Anlage nach einem der vorangehenden Ansprüche, **dadurch gekennzeichnet, dass** das mindestens eine Infrarotspektrometer ausgelegt und eingerichtet ist, den Gehalt an zwei oder mehr Inhaltstoffen, insbesondere Getränkeinhaltstoffen, simultan zu ermitteln, und/oder dass
das mindestens eine Infrarotspektrometer ausgelegt und eingerichtet ist, den mindestens einen Inhaltsstoff in dem fluiden, insbesondere flüssigen, Medium oder in dem wässrigen Lebensmittel quantitativ zu erfassen.

7. Anlage nach einem der vorangehenden Ansprüche, **dadurch gekennzeichnet, dass** das mindestens eine Infrarotspektrometer ausgelegt und eingerichtet ist, den mindestens einen Inhaltsstoff, insbesondere die Vielzahl an Inhaltsstoffen, für wässrige Lebensmittel in wiederkehrenden Messintervallen im Bereich von 5 bis 90 Sekunden, bevorzugt im Bereich von 10 bis 20 Sekunden, zu detektieren.

8. Anlage nach einem der Ansprüche 3 bis 7, ferner umfassend
j) mindestens eine Verschlussvorrichtung, ausgelegt und eingerichtet zum, insbesondere dichten, Verschließen des mit der Abfüllvorrichtung befüllten Gebindegrundkörpers, bevorzugt Getränkegebindegrundkörpers, besonders bevorzugt des befüllten Getränkedosengrundkörpers, mit einem Gebindeverschluss, insbesondere einem Getränkedosendeckel.

9. Anlage nach einem der vorangehenden Ansprüche, **dadurch gekennzeichnet, dass** diese eine Anlage zur High-Speed-Herstellung einer Vielzahl an mit einem wässrigen Lebensmittel, insbesondere Getränke, befüllten Gebinden, insbesondere Getränkegebinden, darstellt.

10. Anlage nach einem der vorangehenden Ansprüche, ferner umfassend mindestens eine Anmischvorrichtung, ausgelegt und eingerichtet zum Mischen von Inhaltsstoffen, insbesondere Getränkeinhaltsstoffen, unter Erhalt eines wässrigen Lebensmittelkonzentrats, insbesondere eines Getränkemischungskonzentrats, enthaltend
einen Anmischbehälter sowie
eine Zuleitung zu diesem Anmischbehälter für ein wässriges System, insbesondere Wasser, und
eine Zuleitung zu diesem Anmischbehälter für mindestens einen ersten Inhaltsstoff, insbesondere Getränkeinhaltsstoff, oder eine erste Inhaltsstoffmischung, insbesondere Getränkeinhaltsstoffmischung, sowie
gegebenenfalls eine Zuleitung zu diesem Anmischbehälter für mindestens einen zweiten Inhaltsstoff, insbesondere zweiten Getränkeinhaltsstoff, oder eine zweite Inhaltsstoffmischung, insbesondere Getränkeinhaltsstoffmischung, sowie gegebenenfalls eine Zuleitung zu diesem Anmischbehälter für mindestens einen dritten Inhaltsstoff, insbesondere Getränkeinhaltsstoff, oder eine dritte Inhaltsstoffmischung, insbesondere Getränkeinhaltsstoffmischung, sowie gegebenenfalls eine Zuleitung zu diesem Anmischbehälter für mindestens einen vierten Inhaltsstoff, insbesondere Getränkeinhaltsstoff, oder eine vierte Inhaltsstoffmischung, insbesondere Getränkeinhaltsstoffmischung, und mindestens eine Transferleitung für ein flüssiges Medium, enthaltend das oder bestehend aus dem in der Anmischvorrichtung gemischten wässrigen Lebensmittelkonzentrat, insbesondere Getränkemischungskonzentrat, und gegebenenfalls mindestens eine Bypass-Leitung, die, insbesondere benachbart zur Anmischvorrichtung, von der Transferleitung ausgeht und die in die Anmischvorrichtung mündet, sowie
mindestens ein Infrarotspektrometer, eingerichtet und ausgelegt zur inline-Detektion von Inhaltsstoffen für wässrige Lebensmittel in der Transferleitung und/oder mindestens ein Infrarotspektrometer, eingerichtet und ausgelegt zur inline-Detektion von Inhaltsstoffen für wässrige Lebensmittel in der Bypass-Leitung.

11. Anlage nach einem der vorangehenden Ansprüche, ferner umfassend
ein Massenspektrometer, bevorzugt ein qTOF-Massenspektrometer und besonders bevorzugt eine ESI-qTOF-Massenspektrometer, enthaltend eine Probenleitung, eingerichtet und ausgelegt zur Detektion von Inhaltsstoffen, insbesondere Getränkeinhaltsstoffen, in wässrigen Lebensmitteln oder in Vorstufen hiervon.

12. Verfahren zur, insbesondere kontinuierlichen, Herstellung von wässrigen Lebensmitteln, insbesondere Getränken, umfassend
die Zurverfügungstellung einer Anlage, insbesondere High-Speed-Produktionsanlage, gemäß einem oder mehreren der vorangehenden Patentansprüche,
die Zurverfügungstellung von Gebindegrundkörpern, bevorzugt Getränkegebindegrundkörpern, besonders bevorzugt Getränkedosengrundkörpern, enthaltend einen Gebindeboden, eine Gebindewandung und eine Einfüllöffnung, die Zurverfügungstellung eines Gebindeverschlusses, insbesondere eines Getränkedosendeckels,
die Zurverfügungstellung eines, insbesondere carbonisierten, wässrigen Systems, die Zurverfügungstellung von mindestens einem, insbesondere einer Vielzahl an Inhaltsstoffen, insbesondere Getränkeinhaltsstoffen, sowie
gegebenenfalls die Zurverfügungstellung von mindestens einem, insbesondere carbonisierten, wässrigen Süßungsmittelkonzentrat,
das Mischen des wässrigen Systems mit dem mindestens einen oder der Vielzahl an Inhaltsstoffen sowie gegebenenfalls dem wässrigen Süßungsmittelkonzentrat in der Mischvorrichtung unter Erhalt eines wässrigen Lebensmittels, insbesondere einer wässrigen Getränkemischung,
das Einfüllen des, insbesondere carbonisierten, wässrigen Lebensmittels in die Vielzahl an Gebindegrundkörpern und
das Verschließen der mit dem wässrigen Lebensmittel befüllten Gebindegrundkörper mit einem Gebindeverschluss mittels der Verschlussvorrichtung,
ferner umfassend
die, insbesondere quantitative, inline-Detektion des mindestens einen Inhaltstoffs in einem in der mindestens einen Zuleitung transportieren fluiden, insbesondere flüssigen, Mediums durch das mindestens eine, dieser Zuleitung zugeordnete FT-NIR-Spektrometer und/oder
die, insbesondere quantitative, inline-Detektion des mindestens einen Inhaltstoffs in der Ableitung für flüssige Medien durch das mindestens eine, dieser Ableitung zugeordnete FT-NIR-Spektrometer und/oder
die, insbesondere quantitative, inline-Detektion des mindestens einen Inhaltstoffs des in der Mischvorrichtung vorliegenden wässrigen Lebensmittels durch das mindestens eine, dieser Mischvorrichtung zugeordnete FT-NIR-Spektrometer, wobei die infrarotspektroskopisch ermittelten, insbesondere quantitativen, Daten der jeweiligen Inhaltsstoffe, insbesondere Getränkeinhaltsstoffe, der fluiden, insbesondere flüssigen, Medien oder des wässrigen Lebensmittels in einer Datenverarbeitungseinrichtung gespeichert werden und
wobei die infrarotspektroskopisch ermittelten Daten der jeweiligen Inhaltsstoffe, insbesondere Getränkeinhaltsstoffe, der fluiden, insbesondere flüssigen, Medien oder des wässrigen Lebensmittels in der Datenverarbeitungseinrichtung dem Messzeitpunkt zugeordnet werden.

13. Verfahren nach Anspruch 12, **dadurch gekennzeichnet, dass**
das wässrige Lebensmittel, insbesondere Getränk, in der Mischvorrichtung und/oder der Ableitung mit dem der Mischvorrichtung bzw. der Ableitung zugeordneten Infrarotspektrometer auf den Gehalt an Zucker, insbesondere Saccharose, Glucose und/oder Fructose, Zitronensäure, Coffein, Taurin und/oder artifiziellen Süßungsmitteln, insbesondere Acesulfam K und/oder Aspartam, analysiert wird und/oder dass
das wässrige System in dem Anmischbehälter und/oder in der Transferleitung von dem Anmischbehälter und/oder in der Bypass-Leitung des Anmischbehälters und/oder der erste und/oder zweite Inhaltsstoff, insbesondere Getränkeinhaltsstoff, oder die erste und/oder zweite Inhaltsstoffmischung, insbesondere Getränkeinhaltsstoffmischung, in der jeweiligen Zuleitung zu dem Anmischbehälter sowie gegebenenfalls der dritte und/oder vierte Inhaltsstoff, insbesondere Getränkeinhaltsstoff, oder die dritte und/oder vierte Inhaltsstoffmischung, insbesondere Getränkeinhaltsstoffmischung, in der jeweiligen Zuleitung zu dem Anmischbehälter mit dem dem Anmischbehälter, der Transferleitung, der Bypass-Leitung bzw. der jeweiligen Zuleitung zugeordneten Infrarotspektrometer auf den Gehalt an einen oder mehreren Inhaltsstoffen, insbesondere Getränkeinhaltsstoffen, analysiert wird.

14. Verfahren nach Anspruch 12 oder 13, **dadurch gekennzeichnet, dass**
die infrarotspektroskopisch ermittelten und einem Messzeitpunkt zugeordneten Daten der jeweiligen Inhaltsstoffe einem in der Abfüllvorrichtung mit dem wässrigen Lebensmittel, insbesondere der wässrigen Getränkemischung, befüllten Gebindegrundkörper oder einer Mehrzahl an aufeinander folgend mit dem wässrigen Lebensmittel, insbesondere der wässrigen Getränkemischung, befüllten Gebindegrundkörpern zugeordnet werden.

15. Verfahren nach Anspruch 12 oder 14, **dadurch gekennzeichnet, dass**
die infrarotspektroskopisch für einen Messzeitpunkt ermittelten Daten der jeweiligen Inhaltsstoffe, insbesondere Getränkeinhaltsstoffe, der fluiden, insbesondere flüssigen, Medien oder des wässrigen Lebensmittels in der Datenverarbeitungseinrichtung mit hinterlegten Soll-Bereichswerten abgeglichen werden.

16. Verfahren nach Anspruch 15, **dadurch gekennzeichnet, dass**
der Zutritt des wässrigen Lebensmittels, insbesondere Getränkemischung, zu der Abfüllvorrichtung mit Hilfe eines, insbesondere mittels automatischer Aktivierung eines, Umlenkventils in der Ableitung, verhindert wird, sobald die infrarotspektroskopisch ermittelten, insbesondere quantitativen, Daten für mindestens einen Inhaltsstoff, insbesondere Getränkeinhaltsstoff, des wässrigen Lebensmittels außerhalb seines hinterlegten Soll-Bereichswerts liegt, so dass das wässrige Lebensmittel nicht in die Abfüllvorrichtung gelangt.

17. Verfahren nach Anspruch 15, **dadurch gekennzeichnet, dass**
ein Umlenkventil in der Ableitung geschlossen bleibt oder wird, solange bzw. sobald die infrarotspektroskopisch ermittelten, insbesondere quantitativen, Daten für sämtliche Inhaltsstoffe, insbesondere Getränkeinhaltsstoffe, des wässrigen Lebensmittels innerhalb ihrer jeweiligen hinterlegten Soll-Bereichswerte liegen, und/oder dass,
sollten die infrarotspektroskopisch ermittelten, insbesondere quantitativen, Daten für einen oder mehrere Inhaltsstoffe, insbesondere Getränkeinhaltsstoffe, des in der mindestens einen Zuleitung transportierten fluiden, insbesondere flüssigen, Mediums unterhalb der hinterlegten Soll-Bereichswerte liegen, man die Konzentration des oder der Inhaltsstoffe in dem fluiden, insbesondere flüssigen, Medium, das in der mindestens einen Zuleitung transportiert wird, erhöht, bis die infrarotspektroskopisch ermittelten Daten innerhalb der hinterlegten Soll-Bereichswerte liegen, und/oder dass,
sollten die infrarotspektroskopisch ermittelten, insbesondere quantitativen, Daten für einen oder mehrere Inhaltsstoffe, insbesondere Getränkeinhaltsstoffe, des in der mindestens einen Zuleitung transportierten fluiden, insbesondere flüssigen, Mediums oberhalb der hinterlegten Soll-Bereichswerte liegen, man die Konzentration des oder der Inhaltsstoffe in dem fluiden, insbesondere flüssigen, Medium, das in der mindestens einen Zuleitung transportiert wird, erniedrigt, bis die infrarotspektroskopisch ermittelten Daten innerhalb der hinterlegten Soll-Bereichswerte liegen.

18. Verfahren nach einem der Ansprüche 12 bis 17, **dadurch gekennzeichnet, dass** die infrarotspektroskopisch ermittelten, insbesondere quantitativen, Daten für einen oder mehrere Inhaltsstoffe, insbesondere Getränkeinhaltsstoffe, einer Charge des über die Transferleitung dem Anmischbehälter entnommenen wässrigen Lebensmittelkonzentrats, insbesondere eines Getränkemischungskonzentrats, abgleicht mit den infrarotspektroskopisch ermittelten, insbesondere quantitativen, Daten für einen oder mehrere Inhaltsstoffe, insbesondere Getränkeinhaltsstoffe, dieser Charge vor oder bei Eintritt in den Mischbehälter der Mischvorrichtung über die Zuleitung.

19. Verwendung eines FT-NIR-Spektrometers, ausgestattet mit den in den Ansprüchen 1 bis 11 und/oder 12 bis 18 genannten Merkmalen, zur, insbesondere quantitativen, Detektion von einem oder mehreren Inhaltsstoffen für wässrige Lebensmittel, insbesondere Getränke, in einer Anlage zur Herstellung einer Vielzahl an mit einem wässrigen Lebensmittel, insbesondere Getränk, befüllten Gebinden, insbesondere Getränkegebinden, oder zur, insbesondere quantitativen, in-line-Überwachung von einem oder mehreren Inhaltsstoffen für wässrige Lebensmittel, insbesondere Getränke, in einer Anlage zur Herstellung einer Vielzahl an mit einem wässrigen Lebensmittel, insbesondere Getränk, befüllten Gebinden, insbesondere Getränkegebinden.

20. Verwendung nach Anspruch 19, **dadurch gekennzeichnet, dass** die Anlage zur Herstellung einer Vielzahl an mit einem wässrigen Lebensmittel befüllten Gebinden, insbesondere Getränkegebinden, eine Anlage zur High-Speed-Herstellung darstellt.

21. Mischvorrichtung für eine Anlage zur Herstellung eines wässrigen Lebensmittels, insbesondere Getränks wie Energy- oder Softdrink, umfassend mindestens einen Mischbehälter,
mindestens eine Zuleitung für fluide, insbesondere flüssige, Medien, enthaltend mindestens einen Inhaltsstoff, insbesondere eine Vielzahl an Inhaltsstoffen, für wässrige Lebensmittel, zu dem Mischbehälter,
eine Ableitung für ein flüssiges Medium, enthaltend das in dem Mischbehälter gemischte wässrige Lebensmittel, aus dem Mischbehälter
und
gegebenenfalls eine Bypass-Leitung, die, insbesondere benachbart zur Mischvorrichtung, von der Ableitung ausgeht, und die förderstromabwärts, insbesondere benachbart zur Abfüllvorrichtung, in die Ableitung mündet,
sowie
mindestens ein FT-NIR-Spektrometer, eingerichtet und ausgelegt zur in-line-Detektion von Inhaltsstoffen für wässrige Lebensmittel in der mindestens einen Zuleitung für fluide, insbesondere flüssige, Medien und/oder mindestens ein FT-NIR-Spektrometer, eingerichtet und ausgelegt zur in-line-Detektion von Inhaltsstoffen für wässrige Lebensmittel in der mindestens einen Ableitung und/oder
mindestens ein FT-NIR-Spektrometer, eingerichtet und ausgelegt zur inline-Detektion von Inhaltsstoffen für wässrige Lebensmittel in der Bypass-Leitung, wobei das mindestens eine FT-NIR-Spektrometer mindestens einen Lichtleiter und eine Transmissionsprozesssonde umfasst, eingerichtet und ausgelegt, um mit dem mindestens einen fluiden, insbesondere flüssigen, Medium, bestehend aus oder enthaltend mindestens einen Inhaltsstoff oder die Vielzahl an Inhaltsstoffen, oder mit dem wässrigen Lebensmittel in Kontakt zu treten.

22. Anmischvorrichtung, ausgelegt und eingerichtet zum Mischen von Inhaltsstoffen, insbesondere Getränkeinhaltsstoffen, unter Erhalt eines wässrigen Lebensmittelkonzentrats, insbesondere eines Getränkemischungskonzentrats, enthaltend
einen Mischbehälter sowie
eine Zuleitung zu diesem ersten Mischbehälter für ein wässriges System, insbesondere Wasser, und
eine Zuleitung zu diesem ersten Mischbehälter für mindestens einen ersten Inhaltsstoff, insbesondere Getränkeinhaltsstoff, oder eine erste Inhaltsstoffmischung, insbesondere Getränkeinhaltsstoffmischung, sowie gegebenenfalls eine Zuleitung zu diesem Mischbehälter für mindestens einen zweiten Inhaltsstoff, insbesondere zweiten Getränkeinhaltsstoff, oder eine zweite Inhaltsstoffmischung, insbesondere Getränkeinhaltsstoffmischung, sowie gegebenenfalls eine Zuleitung zu diesem Mischbehälter für mindestens einen dritten Inhaltsstoff, insbesondere Getränkeinhaltsstoff, oder eine dritte Inhaltsstoffmischung, insbesondere Getränkeinhaltsstoffmischung, sowie gegebenenfalls eine Zuleitung zu diesem Mischbehälter für mindestens einen vierten Inhaltsstoff, insbesondere Getränkeinhaltsstoff, oder eine vierte Inhaltsstoffmischung, insbesondere Getränkeinhaltsstoffmischung, und mindestens eine Ableitung für ein flüssiges Medium, enthaltend das in der Anmischvorrichtung gemischte wässrige Lebensmittelkonzentrat, insbesondere Getränkemischungskonzentrat, und
gegebenenfalls mindestens eine Bypass-Leitung, die, insbesondere benachbart zur Anmischvorrichtung, von der ersten Ableitung ausgeht, und die in der Anmischvorrichtung mündet, sowie
mindestens ein FT-NIR-Spektrometer, eingerichtet und ausgelegt zur inline-Detektion von Inhaltsstoffen für wässrige Lebensmittel in der Ableitung und/oder mindestens ein FT-NIR-Spektrometer, eingerichtet und ausgelegt zur inline-Detektion von Inhaltsstoffen für wässrige Lebensmittel in der ersten Bypass-Leitung,
wobei das mindestens eine FT-NIR-Spektrometer mindestens einen Lichtleiter und eine Transmissionsprozesssonde umfasst, eingerichtet und ausgelegt, um mit dem mindestens einen fluiden, insbesondere flüssigen, Medium, bestehend aus oder enthaltend mindestens einen Inhaltsstoff oder die Vielzahl an Inhaltsstoffen, oder mit dem wässrigen Lebensmittel in Kontakt zu treten.

## Claims

1. A facility for producing a plurality of containers, in particular beverage containers, filled with an aqueous foodstuff, in particular a beverage, comprising
a) at least one mixing device with a mixing container and
b) at least one feed line to the mixing container for fluid media, in particular liquid media, consisting of or containing, in particular containing, at least one ingredient or multiple ingredients, for aqueous foodstuffs,
c) a discharge line from the mixing container for a liquid medium, containing the aqueous foodstuff mixed in the mixing device, and
d) optionally a bypass line, which runs from the discharge line, in particular adjacent to the mixing device, and which opens downstream, in particular adjacent to the filling device, into the discharge line and
e) at least one FT-NIR spectrometer, arranged and adapted for the inline detection of ingredients for aqueous foodstuffs in the at least one feed line for fluid media, and/or
f) at least one FT-NIR spectrometer, arranged and adapted for the inline detection of ingredients for aqueous foodstuffs in the at least one discharge line and/or
g) at least one FT-NIR spectrometer, arranged and adapted for the inline detection of ingredients for aqueous foodstuffs in the bypass line,
wherein the at least one FT-NIR spectrometer comprises at least one light guide and a transmission process probe, arranged and adapted to come into contact with the at least one fluid, in particular liquid, medium, consisting of or containing at least one ingredient or the plurality of ingredients, or with the aqueous foodstuff.

2. The facility according to Claim 1, further comprising
h) at least one infrared spectrometer, arranged and adapted for the inline detection of ingredients for aqueous foodstuffs in the mixing device.

3. The facility according to Claim 1 or 2, further comprising
i) a filling device, which is connected or can be connected to the mixing device via the discharge line, arranged and adapted for filling the aqueous foodstuff, in particular the aqueous beverage mixture, obtained or obtainable by the mixing device, into a plurality of container base bodies, preferably beverage container base bodies, particularly preferably beverage can base bodies, each containing a container bottom, a container wall and a filling opening.

4. The facility according to any of the preceding claims, **characterized by** the discharge line and by the bypass line, which runs from the discharge line, in particular adjacent to the mixing device, and which runs into the discharge line downstream, in particular adjacent to the filling device,
and the measuring cell, which comes into contact with the aqueous foodstuff between the outlet and the opening of the bypass line in the discharge line.

5. The facility according to any of the preceding claims, **characterized in that**
the at least one infrared spectrometer comprises a data processing device, arranged and adapted to store and to evaluate, in particular qualitatively and/or quantitatively, in particular compare with reference data stored in the data processing device for the respective ingredient or ingredients, the infrared spectroscopically determined data of the respective ingredients, in particular beverage ingredients, of the fluid, in particular liquid, media or of the aqueous foodstuff.

6. The facility according to any of the preceding claims, **characterized in that**
the at least one infrared spectrometer is adapted and arranged to simultaneously determine the content of two or more ingredients, in particular beverage ingredients, and/or that the at least one infrared spectrometer is adapted and arranged to determine quantitatively the at least one ingredient in the fluid, in particular liquid, medium or in the aqueous foodstuff.

7. The facility according to any of the preceding claims, **characterized in that**
the at least one infrared spectrometer is adapted and arranged to detect the at least one ingredient, in particular the plurality of ingredients, of aqueous foodstuffs at recurring measurement intervals in the range of 5 to 90 seconds and preferably in the range of 10 to 20 seconds.

8. The facility according to any of claims 3 to 7, further comprising
j) at least one closure device, adapted and arranged for, in particular tightly, closing the container base body filled by the filling device, preferably beverage container base body, particularly preferably the filled beverage can base body, with a container closure, in particular a beverage can lid.

9. The facility according to any of the preceding claims, **characterized in that** said facility is a facility for the high-speed production of a plurality of containers, in particular beverage containers, filled with an aqueous foodstuff, in particular beverage.

10. The facility according to any of the preceding claims, further comprising at least one blending device, adapted and arranged for blending ingredients, in particular beverage ingredients, obtaining an aqueous foodstuff concentrate, in particular a beverage mixture concentrate, containing
a blending container and
a feed line to this blending container for an aqueous system, in particular water, and
a feed line to this blending container for at least one first ingredient, in particular beverage ingredient, or a first ingredient mixture, in particular beverage ingredient mixture, and
optionally a feed line to this blending container for at least one second ingredient, in particular second beverage ingredient, or a second ingredient mixture, in particular beverage ingredient mixture, and optionally a feed line to this blending container for at least one third ingredient, in particular beverage ingredient, or a third ingredient mixture, in particular beverage ingredient mixture, and optionally a feed line to this blending container for at least one fourth ingredient, in particular beverage ingredient, or a fourth ingredient mixture, in particular beverage ingredient mixture, and at least one transfer line for a liquid medium, containing or consisting of the aqueous foodstuff concentrate blended in the blending device, in particular beverage mixture concentrate, and optionally at least one bypass line, which runs from the transfer line, in particular adjacent to the blending device, and which runs into the blending device, and
at least one infrared spectrometer, arranged and adapted for the inline detection of ingredients of aqueous foodstuffs in the transfer line and/or
at least one infrared spectrometer, arranged and adapted for the inline detection of ingredients for aqueous foodstuffs in the bypass line.

11. The facility according to any of the preceding claims, further comprising
a mass spectrometer, preferably a qTOF mass spectrometer and particularly preferably an ESI-qTOF mass spectrometer, containing a sample line, arranged and adapted for the detection of ingredients, in particular beverage ingredients, in aqueous foodstuffs or in preliminary stages thereof.

12. A method for the production, in particular for the continuous production, of aqueous foodstuffs, in particular beverages, comprising providing a facility, in particular a high-speed production facility,
according to one or more of the preceding claims,
providing container base bodies, preferably beverage container base bodies, particularly preferably beverage can base bodies, containing a container bottom, a container wall and a filling opening, providing a container closure, in particular a beverage can lid,
providing an, in particular carbonated, aqueous system, providing at least one, in particular a plurality of ingredients, in particular beverage ingredients, and optionally providing at least one, in particular carbonated, aqueous sweetener concentrate,
mixing the aqueous systems with the at least one or the plurality of ingredients and optionally the aqueous sweetener concentrate in the mixing device obtaining an aqueous foodstuff, in particular an aqueous beverage mixture,
filling the, in particular carbonated, aqueous foodstuff into the plurality of container base bodies and
closing the container base bodies filled with the aqueous foodstuff with a container closure by means of the closure device,
further comprising
the, in particular quantitative, inline detection of the at least one ingredient in a fluid, in particular liquid, medium transported in the at least one feed line by the at least one FT-NIR spectrometer assigned to this feed line and/or
the, in particular quantitative, inline detection of the at least one ingredient in the discharge line for liquid media by the at least one FT-NIR spectrometer assigned to this discharge line and/or
the, in particular quantitative, inline detection of the at least one ingredient of the aqueous foodstuff present in the mixing device by the at least one FT-NIR spectrometer assigned to this mixing device,
wherein the infrared spectroscopically determined, in particular quantitative, data on respective ingredients, in particular beverage ingredients, of the fluid, in particular liquid, media or the aqueous foodstuff are stored on a data processing device and in that the infrared spectroscopically determined data of the respective ingredients, in particular beverage ingredients, of the fluid, in particular liquid, media or the aqueous foodstuff are assigned on the data processing device to the measuring time point.

13. The method according to Claim 12, **characterized in that** the aqueous foodstuff, in particular beverage, in the mixing device and/or the discharge line is analyzed by the infrared spectrometer assigned to the mixing device, or the discharge line for the content of sugar, in particular saccharose, glucose and/or fructose, citric acid, caffeine, taurine and/or artificial sweeteners, in particular acesulfame K and/or aspartame, and/or **in that** the aqueous system in the blending container, and/or in the transfer line from the blending container and/or in the bypass line of the blending container and/or the first and/or second ingredient, in particular beverage ingredient, or the first and/or second ingredient mixture, in particular beverage ingredient mixture, in the respective feed line to the blending container and optionally the third and/or fourth ingredient, in particular beverage ingredient, or the third and/or fourth ingredient mixture, in particular beverage ingredient mixture, in the respective feed line to the blending container is analyzed by the infrared spectrometer assigned to the respective blending container, the respective transfer line, the respective bypass line or the respective feed line for the content of one or more ingredients, in particular beverage ingredients.

14. The method according to Claim 12 or 13, **characterized in that** the infrared spectroscopically determined data on the respective ingredients assigned to a measuring time point are assigned to a container base body filled in the filling device with the aqueous foodstuff, in particular the aqueous beverage mixture, or a plurality of successive container base bodies filled with the aqueous foodstuff, in particular the aqueous beverage mixture.

15. The method according to any of claims 12 to 14, **characterized in that** the infrared spectroscopically determined data of the respective ingredients, in particular beverage ingredients, of the fluid, in particular liquid, media or of the aqueous foodstuff for a measurement time point are compared in the data processing device with stored target value ranges.

16. The method according to claim 15, **characterized in that**
the access of the aqueous foodstuff, in particular beverage mixture, to the filling device is prevented by, in particular by means of an automatic activation of, a diverter valve in the discharge line, as soon as the infrared spectroscopically determined, in particular quantitative, data for at least one ingredient, in particular beverage ingredient, of the aqueous foodstuff is outside its stored target value range, so that the aqueous foodstuff does not enter the filling device.

17. The method according to claim 15, **characterized in that** a diverter valve remains closed or is closed in the discharge line, as long as or as soon as the infrared spectroscopically determined, in particular quantitative, data for all ingredients, in particular beverage ingredients, of the aqueous foodstuff are within their respectively saved target value ranges, and/or **in that**
if the infrared spectroscopically determined, in particular quantitative, data for one or more ingredients, in particular beverage ingredients, of the fluid, in particular liquid, medium transported in the at least one feed line, are below the stored target range values, the concentration of the ingredient(s) in the fluid, in particular liquid, medium, which is transported in the at least one feed line, is increased until the infrared spectroscopically determined data lies within the stored target range values, and/or **in that**,
if the infrared spectroscopically determined, in particular quantitative, data for one or more ingredients, in particular beverage ingredients, of the fluid, in particular liquid, medium transported in the at least one feed line, are above the saved target value ranges, the concentrations of the ingredient or ingredients in the fluid, in particular liquid, medium, which is transported in the at least one feed line, are decreased until the infrared spectroscopically determined data is within the stored target value ranges.

18. The method according to any of claims 12 to 17, **characterized in that** the infrared spectroscopically determined, in particular quantitative, data for one or more ingredients, in particular beverage ingredients, of a batch of the aqueous foodstuff concentrate removed via the transfer line from the blending container, in particular a beverage mixture concentrate, are compared with the infrared spectroscopically determined, in particular quantitative, data for one or more ingredients, in particular beverage ingredients, of this batch before or on entry into the mixing container of the mixing device via the feed line.

19. Use of an FT-NIR spectrometer equipped with the features given in claims 1 to 11 and/or 12 to 18, for the, in particular quantitative, detection of one or more ingredients of aqueous foodstuffs, in particular beverages, in a facility for producing a plurality of containers, in particular beverage containers, filled with an aqueous foodstuff, in particular beverage, or for the, in particular quantitative, inline monitoring of one or more ingredients of aqueous foodstuffs, in particular beverages, in a facility for producing a plurality of containers, in particular beverage containers, filled with an aqueous foodstuff, in particular beverage.

20. Use according to Claim 19, **characterized in that**
the facility for producing a plurality of containers filled with an aqueous foodstuff, in particular beverage containers, is a facility for high-speed production.

21. A mixing device for a facility for producing an aqueous foodstuff, in particular beverage, such as an energy or soft drink, comprising
at least one mixing container,
at least one feed line to the mixing container for fluid, in particular liquid, media, containing at least one ingredient, in particular a plurality of ingredients, of aqueous foodstuffs,
a discharge line from the mixing container for a liquid medium, containing the aqueous foodstuff mixed in the mixing container, and
optionally a bypass line, which runs from the discharge line, in particular adjacent to the mixing device, and which runs downstream, in particular adjacent to the filling device, into the discharge line, and
at least one FT-NIR spectrometer, arranged and adapted for the inline detection of ingredients for aqueous foodstuffs in the at least one feed line for fluid, in particular liquid, media and/or
at least one FT-NIR spectrometer, arranged and adapted for the inline detection of ingredients for aqueous foodstuffs in the at least one discharge line and/or
at least one FT-NIR spectrometer, arranged and adapted for the inline detection of ingredients for aqueous foodstuffs in the bypass line, wherein the at least one FT-NIR spectrometer comprises at least one light guide and a transmission process probe, arranged and adapted to come into contact with the at least one fluid, in particular liquid, medium, consisting of or containing at least one ingredient or the plurality of ingredients, or with the aqueous foodstuff.

22. A blending device, adapted and arranged for mixing ingredients, in particular beverage ingredients, obtaining an aqueous foodstuff concentrate, in particular a beverage mixture concentrate, containing
a mixing container and
a feed line to this first mixing container for an aqueous system, in particular water, and
a feed line to this first mixing container for at least one first ingredient, in particular beverage ingredient, or a first ingredient mixture, in particular beverage ingredient mixture, and
optionally a feed line to this mixing container for at least one second ingredient, in particular second beverage ingredient, or a second ingredient mixture, in particular beverage ingredient mixture, and
optionally a feed line to this mixing container for at least one third ingredient, in particular beverage ingredient, or a third ingredient mixture, in particular beverage ingredient mixture, and
optionally a feed line to this mixing container for at least one fourth ingredient, in particular beverage ingredient, or a fourth ingredient mixture, in particular beverage ingredient mixture, and at least one discharge line containing the aqueous foodstuff concentrate mixed in the blending device, in particular beverage mixture concentrate, and
optionally at least one bypass line, which runs, in particular adjacent to the blending device, from the discharge line or transfer line, and which runs into the blending device, and
at least one FT-NIR spectrometer, arranged and adapted for the inline detection of ingredients of aqueous foodstuffs in the discharge line and/or
at least one FT-NIR spectrometer, arranged and adapted for the inline detection of ingredients for aqueous foodstuffs in the first bypass line, wherein the at least one FT-NIR spectrometer comprises at least one light guide and a transmission process probe, arranged and adapted to come into contact with the at least one fluid, in particular liquid, medium, consisting of or containing at least one ingredient or the plurality of ingredients, or with the aqueous foodstuff.

## Revendications

1. Installation de fabrication d'une pluralité de récipients, en particulier de récipients de boisson, remplis d'un produit alimentaire aqueux, en particulier d'une boisson, comprenant
a) au moins un dispositif de mélange avec un récipient de mélange et
b) au moins une conduite d'alimentation pour des milieux fluides, en particulier des milieux liquides, constitués de ou contenant, en particulier contenant, au moins un ingrédient ou une pluralité d'ingrédients pour des produits alimentaires aqueux, vers le récipient de mélange,
c) une conduite d'évacuation pour un milieu liquide, contenant le produit alimentaire aqueux mélangé dans le dispositif de mélange, hors du récipient de mélange et
d) le cas échéant une conduite de dérivation, qui part de la conduite d'évacuation, en particulier à proximité du dispositif de mélange, et qui débouche dans la conduite d'évacuation en aval du flux de transport, en particulier à proximité du dispositif de remplissage,
ainsi que
e) au moins un spectromètre FT-NIR, aménagé et conçu pour la détection en ligne d'ingrédients pour des produits alimentaires aqueux dans l'au moins une conduite d'alimentation pour des milieux fluides et/ou
f) au moins un spectromètre FT-NIR, aménagé et conçu pour la détection en ligne d'ingrédients pour des produits alimentaires aqueux dans l'au moins une conduite d'évacuation et/ou
g) au moins un spectromètre FT-NIR, aménagé et conçu pour la détection en ligne d'ingrédients pour des produits alimentaires aqueux dans la conduite de dérivation,
dans lequel l'au moins un spectromètre FT-NIR comprend au moins un guide de lumière et une sonde de processus de transmission, aménagé et conçu pour entrer en contact avec l'au moins un milieu fluide, en particulier liquide, constitué de ou contenant au moins un ingrédient ou la pluralité d'ingrédients, ou avec le produit alimentaire aqueux.

2. Installation selon la revendication 1, comprenant en outre
h) au moins un spectromètre infrarouge, aménagé et conçu pour la détection en ligne d'ingrédients pour des produits alimentaires aqueux dans le dispositif de mélange.

3. Installation selon la revendication 1 ou 2, comprenant en outre
i) un dispositif de remplissage, qui est relié ou peut être relié au dispositif de mélange par le biais de la conduite d'évacuation, conçu et aménagé pour le remplissage du produit alimentaire aqueux, en particulier du mélange de boissons aqueux, obtenu ou pouvant être obtenu avec le dispositif de mélange, dans une pluralité de corps de base de récipient, de préférence de corps de base de récipient de boisson, de manière particulièrement préférée de corps de base de récipient de boisson, contenant à chaque fois un fond de récipient, une paroi de récipient et une ouverture de remplissage.

4. Installation selon l'une quelconque des revendications précédentes, **caractérisée par**
la conduite d'évacuation et la conduite de dérivation, qui part de la conduite d'évacuation, en particulier à proximité du dispositif de mélange, et qui débouche dans la conduite d'évacuation en aval du flux de transport, en particulier à proximité du dispositif de remplissage, ainsi qu'une cellule de mesure, qui entre en contact avec le produit alimentaire aqueux entre la sortie et l'embouchure de la conduite de dérivation dans la conduite d'évacuation.

5. Installation selon l'une quelconque des revendications précédentes, **caractérisée en ce que**
l'au moins un spectromètre infrarouge comprend un dispositif de traitement de données, aménagé et conçu pour stocker les données déterminées par spectroscopie infrarouge des ingrédients respectifs, en particulier des ingrédients de boisson, des milieux fluides, en particulier liquides, ou du produit alimentaire aqueux et pour les évaluer, en particulier qualitativement et/ou quantitativement, en particulier pour les mettre en correspondance avec des données de référence stockées dans le dispositif de traitement de données pour le ou les ingrédients respectifs.

6. Installation selon l'une quelconque des revendications précédentes, **caractérisée en ce que**
l'au moins un spectromètre infrarouge est conçu et aménagé pour déterminer simultanément la teneur en deux substances de contenu ou plus, en particulier en substances de contenu de boisson, et/ou **en ce que**
l'au moins un spectromètre infrarouge est conçu et aménagé pour détecter quantitativement l'au moins un ingrédient dans le milieu fluide, en particulier liquide, ou dans le produit alimentaire aqueux.

7. Installation selon l'une quelconque des revendications précédentes, **caractérisée en ce que**
l'au moins un spectromètre infrarouge est conçu et aménagé pour détecter l'au moins un ingrédient, en particulier la pluralité d'ingrédients, pour des produits alimentaires aqueux à des intervalles de mesure récurrents dans la plage de 5 à 90 secondes, de préférence dans la plage de 10 à 20 secondes.

8. Installation selon l'une quelconque des revendications 3 à 7, comprenant en outre
j) au moins un dispositif de fermeture, conçu et aménagé pour la fermeture, en particulier étanche, du corps de base de récipient rempli avec le dispositif de remplissage, de préférence du corps de base de récipient de boisson, de manière particulièrement préférée du corps de base de récipient de boisson rempli, avec une fermeture de récipient, en particulier un couvercle de récipient de boisson.

9. Installation selon l'une quelconque des revendications précédentes, **caractérisée en ce que**
celle-ci représente une installation de fabrication à grande vitesse d'une pluralité de récipients, en particulier de récipients de boisson, remplis d'un produit alimentaire aqueux, en particulier de boissons.

10. Installation selon l'une quelconque des revendications précédentes, comprenant en outre
au moins un dispositif de mélange, conçu et aménagé pour le mélange d'ingrédients, en particulier d'ingrédients de boisson, avec obtention d'un concentré de produit alimentaire aqueux, en particulier d'un concentré de mélange de boissons, contenant
un récipient de mélange ainsi que
une conduite d'alimentation vers ce récipient de mélange pour un système aqueux, en particulier de l'eau, et
une conduite d'alimentation vers ce récipient de mélange pour au moins un premier ingrédient, en particulier un ingrédient de boisson, ou un premier mélange d'ingrédients, en particulier un mélange d'ingrédients de boisson, ainsi que le cas échéant une conduite d'alimentation vers ce récipient de mélange pour au moins un deuxième ingrédient, en particulier un deuxième ingrédient de boisson, ou un deuxième mélange d'ingrédients, en particulier un mélange d'ingrédients de boisson, ainsi que
le cas échéant une conduite d'alimentation vers ce récipient de mélange pour au moins un troisième ingrédient, en particulier un ingrédient de boisson, ou un troisième mélange d'ingrédients, en particulier un mélange d'ingrédients de boisson, ainsi que
le cas échéant une conduite d'alimentation vers ce récipient de mélange pour au moins un quatrième ingrédient, en particulier un ingrédient de boisson, ou un quatrième mélange d'ingrédients, en particulier un mélange d'ingrédients de boisson, et
au moins une conduite de transfert pour un milieu liquide, contenant ou constitué du concentré de produit alimentaire aqueux, en particulier du concentré de mélange de boissons, mélangé dans le dispositif de mélange, et
le cas échéant au moins une conduite de dérivation, qui part de la conduite de transfert, en particulier à proximité du dispositif de mélange, et qui débouche dans le dispositif de mélange, ainsi que
au moins un spectromètre infrarouge, aménagé et conçu pour la détection en ligne d'ingrédients pour des produits alimentaires aqueux dans la conduite de transfert et/ou
au moins un spectromètre infrarouge, aménagé et conçu pour la détection en ligne d'ingrédients pour des produits alimentaires aqueux dans la conduite de dérivation.

11. Installation selon l'une quelconque des revendications précédentes, comprenant en outre
un spectromètre de masse, de préférence un spectromètre de masse qTOF et de manière particulièrement préférée un spectromètre de masse ESI qTOF, contenant une conduite d'échantillon, aménagé et conçu pour la détection d'ingrédients, en particulier d'ingrédients de boisson, dans des produits alimentaires aqueux ou dans des précurseurs de ceux-ci.

12. Procédé de fabrication, en particulier en continu, de produits alimentaires aqueux, en particulier de boissons, comprenant
la mise à disposition d'une installation, en particulier d'une installation de production à grande vitesse, selon une ou plusieurs des revendications précédentes,
la mise à disposition de corps de base de récipient, de préférence de corps de base de récipient de boisson, de manière particulièrement préférée de corps de base de récipient de boisson, contenant un fond de récipient, une paroi de récipient et une ouverture de remplissage,
la mise à disposition d'une fermeture de récipient, en particulier d'un couvercle de récipient de boisson,
la mise à disposition d'un système aqueux, en particulier carbonisé,
la mise à disposition d'au moins un, en particulier d'une pluralité d'ingrédients, en particulier d'ingrédients de boisson, ainsi que
le cas échéant la mise à disposition d'au moins un concentré d'édulcorant aqueux, en particulier carbonisé,
le mélange du système aqueux avec l'au moins un ou la pluralité d'ingrédients ainsi que le cas échéant le concentré d'édulcorant aqueux dans le dispositif de mélange avec obtention d'un produit alimentaire aqueux, en particulier d'un mélange de boissons aqueux,
le remplissage du produit alimentaire aqueux, en particulier carbonisé, dans la pluralité de corps de base de récipient et
la fermeture du corps de base de récipient rempli du produit alimentaire aqueux avec une fermeture de récipient au moyen du dispositif de fermeture, comprenant en outre
la détection, en particulier quantitative, en ligne de l'au moins une substance de contenu dans un milieu fluide, en particulier liquide, transporté dans l'au moins une conduite d'alimentation par l'au moins un spectromètre FT-NIR associé à cette conduite d'alimentation et/ou
la détection, en particulier quantitative, en ligne de l'au moins une substance de contenu dans la conduite d'évacuation pour des milieux liquides par l'au moins un spectromètre FT-NIR associé à cette conduite d'évacuation et/ou
la détection, en particulier quantitative, en ligne de l'au moins une substance de contenu du produit alimentaire aqueux présent dans le dispositif de mélange par l'au moins un spectromètre FT-NIR associé à ce dispositif de mélange,
dans lequel les données déterminées par spectroscopie infrarouge, en particulier quantitatives, des ingrédients respectifs, en particulier des ingrédients de boisson, des milieux fluides, en particulier liquides, ou du produit alimentaire aqueux sont stockées dans un dispositif de traitement de données et
dans lequel les données déterminées par spectroscopie infrarouge des ingrédients respectifs, en particulier des ingrédients de boisson, des milieux fluides, en particulier liquides, ou du produit alimentaire aqueux sont associées au moment de mesure dans le dispositif de traitement de données.

13. Procédé selon la revendication 12, **caractérisé en ce que**
le produit alimentaire aqueux, en particulier la boisson, est analysé dans le dispositif de mélange et/ou la conduite d'évacuation avec le spectromètre infrarouge associé au dispositif de mélange ou à la conduite d'évacuation pour la teneur en sucre, en particulier saccharose, glucose et/ou fructose, acide citrique, caféine, taurine et/ou édulcorants artificiels, en particulier acésulfame K et/ou aspartame, et/ou **en ce que** le système aqueux est analysé dans le récipient de mélange et/ou dans la conduite de transfert du récipient de mélange et/ou dans la conduite de dérivation du récipient de mélange et/ou le premier et/ou deuxième ingrédient, en particulier ingrédient de boisson, ou le premier et/ou deuxième mélange d'ingrédients, en particulier mélange d'ingrédients de boisson, dans la conduite d'alimentation respective vers le récipient de mélange ainsi que le cas échéant le troisième et/ou quatrième ingrédient, en particulier ingrédient de boisson, ou le troisième et/ou quatrième mélange d'ingrédients, en particulier mélange d'ingrédients de boisson, dans la conduite d'alimentation respective vers le récipient de mélange avec le spectromètre infrarouge associé au récipient de mélange, à la conduite de transfert, à la conduite de dérivation ou à la conduite d'alimentation respective pour la teneur en un ou plusieurs ingrédients, en particulier ingrédients de boisson.

14. Procédé selon la revendication 12 ou 13, **caractérisé en ce que**
les données déterminées par spectroscopie infrarouge et associées à un moment de mesure des ingrédients respectifs sont associées à un corps de base de récipient rempli dans le dispositif de remplissage avec le produit alimentaire aqueux, en particulier le mélange de boissons aqueux, ou à une pluralité de corps de base de récipient remplis successivement avec le produit alimentaire aqueux, en particulier le mélange de boissons aqueux.

15. Procédé selon la revendication 12 ou 14, **caractérisé en ce que**
les données déterminées par spectroscopie infrarouge pour un moment de mesure des ingrédients respectifs, en particulier des ingrédients de boisson, des milieux fluides, en particulier liquides, ou du produit alimentaire aqueux sont mises en correspondance avec des valeurs de plage de consigne stockées dans le dispositif de traitement de données.

16. Procédé selon la revendication 15, **caractérisé en ce que**
l'accès du produit alimentaire aqueux, en particulier du mélange de boissons, au dispositif de remplissage est empêché à l'aide d'une vanne de déviation, en particulier au moyen d'une activation automatique d'une vanne de déviation dans la conduite d'évacuation, dès que les données déterminées par spectroscopie infrarouge, en particulier quantitatives, pour au moins un ingrédient, en particulier un ingrédient de boisson, du produit alimentaire aqueux se trouvent en dehors de sa valeur de plage de consigne stockée, de sorte que le produit alimentaire aqueux ne parvient pas dans le dispositif de remplissage.

17. Procédé selon la revendication 15, **caractérisé en ce que**
une vanne de déviation reste ou est fermée dans la conduite d'évacuation tant que ou dès que les données déterminées par spectroscopie infrarouge, en particulier quantitatives, pour tous les ingrédients, en particulier les ingrédients de boisson, du produit alimentaire aqueux se trouvent dans leurs valeurs de plage de consigne stockées respectives, et/ou **en ce que**
si les données déterminées par spectroscopie infrarouge, en particulier quantitatives, pour un ou plusieurs ingrédients, en particulier des ingrédients de boisson, du milieu fluide, en particulier liquide, transporté dans l'au moins une conduite d'alimentation se trouvent en dessous des valeurs de plage de consigne stockées, la concentration du ou des ingrédients dans le milieu fluide, en particulier liquide, qui est transporté dans l'au moins une conduite d'alimentation est augmentée jusqu'à ce que les données déterminées par spectroscopie infrarouge se trouvent dans les valeurs de plage de consigne stockées, et/ou **en ce que** si les données déterminées par spectroscopie infrarouge, en particulier quantitatives, pour un ou plusieurs ingrédients, en particulier des ingrédients de boisson, du milieu fluide, en particulier liquide, transporté dans l'au moins une conduite d'alimentation se trouvent au-dessus des valeurs de plage de consigne stockées, la concentration du ou des ingrédients dans le milieu fluide, en particulier liquide, qui est transporté dans l'au moins une conduite d'alimentation est diminuée jusqu'à ce que les données déterminées par spectroscopie infrarouge se trouvent dans les valeurs de plage de consigne stockées.

18. Procédé selon l'une quelconque des revendications 12 à 17, **caractérisé en ce que** les données déterminées par spectroscopie infrarouge, en particulier quantitatives, pour un ou plusieurs ingrédients, en particulier des ingrédients de boisson, d'un lot du concentré de produit alimentaire aqueux, en particulier d'un concentré de mélange de boissons, prélevé du récipient de mélange par le biais de la conduite de transfert sont comparées aux données déterminées par spectroscopie infrarouge, en particulier quantitatives, pour un ou plusieurs ingrédients, en particulier des ingrédients de boisson, de ce lot avant ou lors de l'entrée dans le récipient de mélange du dispositif de mélange par le biais de la conduite d'alimentation.

19. Utilisation d'un spectromètre FT-NIR, équipé des caractéristiques mentionnées dans les revendications 1 à 11 et/ou 12 à 18, pour la détection, en particulier quantitative, d'un ou de plusieurs ingrédients pour des produits alimentaires aqueux, en particulier des boissons, dans une installation de fabrication d'une pluralité de récipients, en particulier de récipients de boisson, remplis d'un produit alimentaire aqueux, en particulier de boissons, ou pour la surveillance, en particulier quantitative, en ligne, d'un ou de plusieurs ingrédients pour des produits alimentaires aqueux, en particulier des boissons, dans une installation de fabrication d'une pluralité de récipients, en particulier de récipients de boisson, remplis d'un produit alimentaire aqueux, en particulier de boissons.

20. Utilisation selon la revendication 19, **caractérisée en ce que** l'installation de fabrication d'une pluralité de récipients, en particulier de récipients de boisson, remplis d'un produit alimentaire aqueux représente une installation de fabrication à grande vitesse.

21. Dispositif de mélange pour une installation de fabrication d'un produit alimentaire aqueux, en particulier d'une boisson telle qu'une boisson énergisante ou une boisson soft, comprenant
au moins un récipient de mélange,
au moins une conduite d'alimentation pour des milieux fluides, en particulier liquides, contenant au moins un ingrédient, en particulier une pluralité d'ingrédients, pour des produits alimentaires aqueux, vers le récipient de mélange,
une conduite d'évacuation pour un milieu liquide, contenant le produit alimentaire aqueux mélangé dans le récipient de mélange, hors du récipient de mélange et et
le cas échéant une conduite de dérivation, qui part de la conduite d'évacuation, en particulier à proximité du dispositif de mélange, et qui débouche dans la conduite d'évacuation en aval du flux de transport, en particulier à proximité du dispositif de remplissage,
ainsi que
au moins un spectromètre FT-NIR, aménagé et conçu pour la détection en ligne d'ingrédients pour des produits alimentaires aqueux dans l'au moins une conduite d'alimentation pour des milieux fluides, en particulier liquides et/ou au moins un spectromètre FT-NIR, aménagé et conçu pour la détection en ligne d'ingrédients pour des produits alimentaires aqueux dans l'au moins une conduite d'évacuation et/ou
au moins un spectromètre FT-NIR, aménagé et conçu pour la détection en ligne d'ingrédients pour des produits alimentaires aqueux dans la conduite de dérivation, dans lequel l'au moins un spectromètre FT-NIR comprend au moins un guide de lumière et une sonde de processus de transmission, aménagé et conçu pour entrer en contact avec l'au moins un milieu fluide, en particulier liquide, constitué de ou contenant au moins un ingrédient ou la pluralité d'ingrédients, ou avec le produit alimentaire aqueux.

22. Dispositif de mélange, conçu et aménagé pour le mélange d'ingrédients, en particulier d'ingrédients de boisson, avec obtention d'un concentré de produit alimentaire aqueux, en particulier d'un concentré de mélange de boissons, contenant
un récipient de mélange ainsi que
une conduite d'alimentation vers ce premier récipient de mélange pour un système aqueux, en particulier de l'eau, et
une conduite d'alimentation vers ce premier récipient de mélange pour au moins un premier ingrédient, en particulier un ingrédient de boisson, ou un premier mélange d'ingrédients, en particulier un mélange d'ingrédients de boisson, ainsi que le cas échéant une conduite d'alimentation vers ce récipient de mélange pour au moins un deuxième ingrédient, en particulier un deuxième ingrédient de boisson, ou un deuxième mélange d'ingrédients, en particulier un mélange d'ingrédients de boisson, ainsi que
le cas échéant une conduite d'alimentation vers ce récipient de mélange pour au moins un troisième ingrédient, en particulier un ingrédient de boisson, ou un troisième mélange d'ingrédients, en particulier un mélange d'ingrédients de boisson, ainsi que
le cas échéant une conduite d'alimentation vers ce récipient de mélange pour au moins un quatrième ingrédient, en particulier un ingrédient de boisson, ou un quatrième mélange d'ingrédients, en particulier un mélange d'ingrédients de boisson, et
au moins une conduite d'évacuation pour un milieu liquide, contenant le concentré de produit alimentaire aqueux, en particulier le concentré de mélange de boissons, mélangé dans le dispositif de mélange, et
le cas échéant au moins une conduite de dérivation, qui part de la première conduite d'évacuation, en particulier à proximité du dispositif de mélange, et qui débouche dans le dispositif de mélange, ainsi que
au moins un spectromètre FT-NIR, aménagé et conçu pour la détection en ligne d'ingrédients pour des produits alimentaires aqueux dans la conduite d'évacuation et/ou
au moins un spectromètre FT-NIR, aménagé et conçu pour la détection en ligne d'ingrédients pour des produits alimentaires aqueux dans la première conduite de dérivation,
dans lequel l'au moins un spectromètre FT-NIR comprend au moins un guide de lumière et une sonde de processus de transmission, aménagé et conçu pour entrer en contact avec l'au moins un milieu fluide, en particulier liquide, constitué de ou contenant au moins un ingrédient ou la pluralité d'ingrédients, ou avec le produit alimentaire aqueux.
